Europäisches Patentamt

European Patent Office

Office européen des brevets

(19)

(11) Veröffentlichungsnummer: **0 234 328**
**B1**

(12) **EUROPÄISCHE PATENTSCHRIFT**

(45) Veröffentlichungstag der Patentschrift:
28.03.90

(51) Int. Cl.⁴: **A61K 31/05**, A61K 31/17,
A61K 31/045

(21) Anmeldenummer: 87101291.0

(22) Anmeldetag: 30.01.87

(54) Abwaschbare topische Zubereitung zur Therapie der Psoriasis.

(30) Priorität: 07.02.86 DE 3603859

(43) Veröffentlichungstag der Anmeldung:
02.09.87 Patentblatt 87/36

(45) Bekanntmachung des Hinweises auf die Patenterteilung:
28.03.90 Patentblatt 90/13

(84) Benannte Vertragsstaaten:
AT BE CH DE ES FR GB IT LI LU NL

(56) Entgegenhaltungen:
DD-A- 236 259
FR-A- 2 396 548
GB-A- 2 113 547
GB-A- 2 157 173

ULLMANNS ENCYKLOPäDIE DER TECHNISCHEN
CHEMIE, Band 22, 4. Auflage, VERLAG CHEMIE,
Seite 489

(73) Patentinhaber: Röhm Pharma GmbH,
Dr.-Otto-Röhm-Strasse 2-4, D-6108 Weiterstadt 1(DE)

(72) Erfinder: Rölz, Wolfgang, Dr., Carl-Schurz-Strasse 55,
D-6070 Langen-Neurott(DE)
Erfinder: Müller, Josef, Dr., Jugendpfad 5,
D-6145 Lindenfels(DE)

## Beschreibung

Die Erfindung betrifft pharmazeutische Zubereitungen zur Behandlung von Psoriasis mit Wirkstoffen aus einer Kombination von Substanzen auf der Basis von 1,8,9-Anthracentriol und Harnstoff, insbesondere auch zur Behandlung der Kopfpsoriasis oder anderer behaarter Körperpartien nach der Methode der Kurzzeittherapie.

Die Psoriasis gehört zu den Dermatosen mit bisher ungeklärter Ätiologie. Im wesentlichen ist man auf die lokale Applikation von Wirkstoffen wie Salicylsäure, Teerarten (pices Var.), Vitamin-A-Säure, Corticosteroiden u.ä. angewiesen. Zum Teil behandelt man auch mit Steinkohlenteer.

Zu den seit langem gegen Psoriasis verwendeten Wirkstoffen gehört das 1,8,9-Anthracentriol, auch als Dithranol oder als Anthralin bezeichnet, sowie bestimmte Acyl-Derivate desselben, meist in 0,1- 5-prozentigen Zubereitungen.

Die Wirkstoffe stehen, bedingt durch ihre hohe Instabilität gegen Luftsauerstoff, Wasser und Alkalien, durchwegs nur in lipophilen pharmazeutischen Darreichungsformen mit beschränkter Lagerstabilität zur lokalen Therapie zur Verfügung. Die Anwendung dieser stark fettenden therapeutischen Zubereitungen kann im allgemeinen nur auf sehr unbequeme Weise erfolgen, da Kleidungsstücke, die auf der therapierten Haut getragen werden, oder Wäschestücke, bedingt durch die Farbreaktionen des Wirkstoffes Dithranol starke, schwer entfernbare Verfärbungen zeigen und die hydrophoben Zubereitungen nur schwer von der Haut und insbesondere aus behaarten Hautarealen abwaschbar sind.

Die Anwendung der üblichen, ursprünglich für die Behandlung unbehaarter Hautpartien vorgesehenen Zubereitungen bereitet beim Psoriasis-Patienten im behaarten Hautbereich in mehrfacher Hinsicht Beschwerden. Die Salbenmasse Vaseline, mit 2 - 3 % Salicylsäure und 0,5 - 3 % Dithranol, als Träger der genannten Wirkstoffe muß durch kräftiges Einreiben in Kontakt z.B. mit der Kopfhaut gebracht werden. Diese Behandlung verursacht bei der bereits vorgeschädigten Psoriatikerhaut in erheblichem Maße Schmerzen. Die Behandlung muß wegen der Komplikationen noch häufig stationär erfolgen.

Eine optimale therapeutische Zubereitungsform zur Behandlung der Psoriasis sollte nach EP-A 6 724 ein Zusammenspiel der Einzelwirkungen des Harnstoffs und des Dithranols ermöglichen. Wahrscheinlich ist, daß die antipsoriatische Wirkung nicht nur vom Dithranol ausgeht. Es muß aufgrund von klinischen Untersuchungen angenommen werden, daß auch der Harnstoff durch seine vielfältigen und bekannten Eigenschaften wie Keratolyse, Hornschichthydratation, die Strukturfunktion der Epidermis so beeinflußt, daß eine positive Auswirkung auf die gestörte Epithelbildung bei Psoriasis zu erwarten ist.

Ein weiterer wichtiger Aspekt bei der Behandlung der Psoriasis ist die Ablösung von Schuppen und die Durchfeuchtung der ausgetrockneten Hornschicht. Hierbei kommt dem Harnstoff eine besondere Bedeutung zu, da nach T.-M. Ernst, Z. Hautkrankheiten (1981) 56, 18: 1197-1206, durch den keratolytischen Effekt die Abstoßung von Schuppen erleichtert wird. Darüber hinaus hat der Harnstoff hydratisierende Eigenschaften, die die Hornschicht zu einer besseren Wasseraufnahme befähigen, so daß im Stratum corneum ein erhöhter Feuchtigkeitsgehalt erzielt wird, der besonders beim Psoriatiker eine wesentliche Verbesserung der Krankheitssymptome bewirkt.

Untersuchungen zur Penetrationskinetik von Dithranol haben gezeigt, daß die Penetrationsgeschwindigkeit an geschädigter Haut beim Psoriatiker, im Vergleich zu gesunder Haut, wesentlich erhöht ist. Aufgrund dieses Sachverhaltes ergibt sich bei der Lokaltherapie der Psoriasis mit dithranolhaltigen Präparaten die Möglichkeit, nach kurzer Einwirkungszeit dieser Zubereitung durch Entfernen des Überstandes die penetrierende Dithranolmenge an gesunder Haut wesentlich zu verringern. Damit werden die Nebenwirkungen, wie starke Hautreizung und Rötung auf gesunder Haut, die häufig zum Therapieabbruch führen, weitgehend vermieden. Die Therapieerfolge dieser nach U. Runne und J. Kunze, Brit. Journ. of Dermatology (1982) 106, 135-139 und Der Hautarzt (1985) 36: 44-46, genannten Minutentherapie werden genauso hoch eingeschätzt wie bei der klassischen Langzeitanwendung von Dithranolpräparaten.

### Aufgabe und Lösung

Voraussetzung für das vorstehend beschriebene Therapieverfahren (Minutentherapie) ist die gute Entfernbarkeit der verwendeten Zubereitung. In dieser Hinsicht gibt es derzeit keine geeignete, stabile Zubereitung mit handelsüblicher Laufzeit, die sich rasch entfernen läßt. So verwendet man sogar eine zweite indifferente Creme zur Aufnahme des Wirkstoffes und entfernt diese mit Detergentien. Es besteht daher zur breiten Anwendung der Minutentherapie zur Behandlung der Psoriasis insbesondere auch im Bereich behaarter Körperregionen, ein Bedarf nach einer topischen Zubereitung, die sich zeitlich genau kontrolliert durch einfaches Abspülen entfernen läßt.

Zur Lösung der geschilderten Aufgabe wird eine Kombination zweier Wirkstoffe mit synergistischem Effekt angewendet.

Die erfindungsgemäße Kombination besteht aus den wirksamen Komponenten

(A) 1,8,9-Anthracentriol und/oder dessen Acyl-Derivaten
(B) Harnstoff in einer für die topische Therapie geeigneten feinkristallinen Form, im folgenden "mikrodisperser Harnstoff" genannt.

Weiterhin sind diese neuartigen Verabreichungsformen dadurch gekennzeichnet, daß sie zu ihrer schnellen Entfernbarkeit vom Applikationsort nach Ende der kurzzeitigen, kontrollierten Einwirkungsdauer sehr leicht durch Waschen entfernt werden können. Hierzu ist, wendet man z.B. Lipidsysteme an, ein zum Teil hoher Gehalt an Tensidsubstanzen notwendig. Viele Tenside, oxethylierte und ionische, zeichnen sich allerdings dadurch aus, daß sie sowohl mit Dithranol als auch mit Harnstoff unverträglich sind (DAC, 1979) und keine lagerfähigen Produkte ergeben.

Erfindungsgemäß wird die destabilisierende Wirkung auf Dithranol verhindert, die Tenside besonders in Gegenwart von Wasser bei ungeeigneter, insbesondere alkalischer pH-Einstellung zeigen, wenn die Tenside und waschaktiven Substanzen, die überwiegend lineare polare Moleküle darstellen, als Harnstoff-Einschlußverbindungen den Zubereitungen zugesetzt werden. Deren Herstellung kann in an sich bekannter Weise sowohl in flüssiger Phase als auch durch geeignete Festkörperreaktionen erfolgen. Die Tenside, die üblicherweise in wäßrigen Pasten oder wasserhaltigen, hochviskosen Flüssigkeiten zur Verfügung stehen, werden dabei in eine wasserfreie Form überführt und stellen so eine stabile, wasserfreie pharmazeutische Darreichungsform dar.

Nach einer gegenüber den bisherigen bekannten Produkten kurzen, kontrollierten therapeutischen Einwirkzeit kann nun vorteilhafterweise durch Wasseranwendung, wie einfaches Abspülen unter äußerst schonender Massage, die Zubereitung restlos entfernt werden. Unter Zusatz von Wasser bildet sich nämlich hier eine Öl-in-Wasser-Emulsion, die mit Wasser verdünnbar, also leicht entfernbar ist. Auch nach Anwendung an behaarten Körperpartien ist das Medikament ohne zusätzlichen Einsatz von Shampoos entfernbar. Besonders hervorzuheben ist hierbei die damit verbundene ausgezeichnete Entfernbarkeit der übermäßig gebildeten Hautschuppen, ein Erfolg der Kombination Tensid und keratolytisch wirkendem Harnstoff.

### Vorteilhafte Wirkungen

Die erfindungsgemäßen therapeutischen Zubereitungen zeichnen sich dadurch aus, daß bei ihrer Applikation auf kräftiges Einreiben in die auf Berührung empfindlichen psoriatischen Hautpartien verzichtet werden kann. Dies ist besonders vorteilhaft bei der Applikation auf der Kopfhaut bzw. anderen behaarten Körperpartien. Wegen der als selbstemulgierend zu charakterisierenden Zubereitungsart sind die erfindungsgemäßen Zubereitungen in der Regel sehr leicht durch Auswaschen vom Applikationsort entfernbar.

Von besonderer Bedeutung ist daher die Tatsache, daß es durch die besondere Verfahrensweise und Zusammensetzung der erfindungsgemäßen therapeutischen Zubereitungen gelingt, die oxidations- und hydrolyseempfindlichen Wirkstoffe Dithranol bzw. dessen Acyl-Derivate und Harnstoff in einer Applikationsform mit hohem hydrophilen Tensidgehalt in einer Zubereitung mit marktüblicher Haltbarkeit zu vereinen. Die Stabilität des Präparates zu marktüblicher Haltbarkeit bei einfachen Lagerbedingungen und ohne Kühllagerung wird hierbei ohne Salicylsäurezusatz erreicht.

Besonders hervorzuheben ist, daß die Therapieform durch die Zubereitung ermöglicht wird und diese weder vor Gebrauch durch den Patienten aus zwei Vorprodukten zum Anwendungsprodukt zubereitet werden muß, wie dies in DE-A 33 02 739 beschrieben ist, noch nach Anwendung durch Verwendung mehrerer Präparate entfernt werden muß.

### Durchführung der Erfindung

Als spezifische, erfindungsgemäße, wasserfreie Verabreichungsformen seien genannt:

a) abwaschbare Salben
b) Waschcremes, Ölhaarkuren
c) hydrophile, selbstemulgierende Ölzubereitungen
d) hydrophiles Reinigungsöl
e) Massageöl/-Creme, abwaschbar
f) Ölschaumbad wasserfrei

Die Technologie der Herstellung solcher Zubereitungen ist ausführlich in Ullmanns Encyklopädie der technischen Chemie, 3. Auflage, 4. Band, Seiten 20 bis 23 und 4. Auflage, Band 10, Seiten 31 bis 39 und 4. Auflage, Band 12, Seiten 557 bis 566, beschrieben.

Die erfindungsgemäßen therapeutischen Zubereitungen enthalten die Komponente A, in der Regel mit einem Anteil von 0,1 bis 5 Gew.-%, vorzugsweise 0,5 bis 3 Gew.-%. Die Komponenten A und B stehen in den erfindungsgemäßen Zubereitungen im allgemeinen im Gewichtsverhältnis von 1 : 1 bis 1 : 100, vorzugsweise 1 : 2 bis 1 : 25.

Als Konsistenzbildner der in ihrer Zähigkeit verschieden eingestellten Verabreichungsformen, kommen chemisch verschiedenartige Komponenten, wie ölige bis wachsartige Kohlenwasserstoffe, beispielsweise Paraffinöle, Polyäthylene mit Molekulargewichten bis max. 1 000, oder Fettsäureester mit langkettigen Alkoholen z.B. Ölsäureoleylester (Cetiol®) oder auch Alkoholen mit beispielsweise 1 bis 4 Kohlenstoffatomen von insbesondere längerkettigen Fettsäuren, oder solche mit insbesondere höheren Fettsäuren, wie z.B. Myristinsäure, Ölsäure, veresterte mehrwertige Alkohole, wie Glycerin, Sorbitol, und langkettige Fettalkohole, wie beispielsweise Myristilalkohol in Betracht. In diese Stoffe, die etwa 70 bis 90 Gew.-% der pharmazeutischen Zubereitung ausmachen, werden die Wirksubstanzen, das Dithranol oder Acyl-Derivate des 1,8,9-Anthracentriols und die Tensid-Harnstoff-Einschlußverbindung nach bekannten Verfahren eingearbeitet. Weitere dermatologisch wirkende Stoffe können gegebenenfalls mitverwendet werden.

Die Herstellung der Tensid-Harnstoff-Einschlußverbindung wird gesondert durchgeführt. Dies kann nach verschiedenen zur Herstellung von Harnstoff-Einschlußverbindungen bekannten Verfahren, wie z.B. durch Zusammengeben von methanolischen und/oder wäßrigen Tensidlösungen mit Harnstoff und durch anschließende Kristallisation, oder ohne Lösungsmittelzusatz durch geeignete Festkörperreaktion, wie inniges Vermischen, z.B. durch Mahlen, ausgeführt werden.

Als Tenside, die mit Harnstoff eine Einschlußverbindung bilden, kommen erfindungsgemäß die wasserlöslichen, und die nach therapeutischer Applikation durch Wasser aus den Einschlußverbindungen freigesetzten und dabei eine Öl-Wasser-Emulsion bildenden, praktisch geradkettigen Oxethylate, d.h. nichtionische Verbindungen, mit HLB-Werten von etwa 8 bis 15 in Frage (zu dem Begriff HLB-Wert und nichtionische Tenside siehe Ullmanns Encyklopädie der technischen Chemie, 4. Auflage, Band 22, Seite 488ff). Solche Verbindungen sind beispielsweise Polyoxyethylen-(20)cetylstearylether, auch als Ceteareth-20 bezeichnet wird (CTFA-Bezeichnung), -CTFA: The Cosmetic, Toiletry and Fragrance Association, Washington - oder Ethylenglykolmonolaurylether mit 2 Ethylenoxy-Einheiten, CTFA-Bezeichnung Laureth-2. Diese Tenside bilden mit Harnstoff Einschlußverbindungen unter Ausbildung der hexagonalen Harnstoff-Struktur, wie röntgenographische Untersuchungen zeigen. Oxethylierte Tenside mit mehreren langen, geradkettigen Resten, die aber von einem Molekülrest, z.B. einem Sorbitanrest abzweigen, zeigen mit Harnstoff kombiniert auch die erfindungsgemäßen Eigenschaften, jedoch sind diese Tensid-Harnstoff-Addukte röntgenamorph. Der Aufbau dieser Addukte ist nicht geklärt. Möglicherweise sind hier die langen, geradkettigen Molekülteile von Harnstoff eingeschlossen, während die voluminöseren Teile, insbesondere die Verzweigungsstellen keine Harnstoffaddukte bilden. Zu diesen Tensiden gehören beispielsweise Polyoxyethylen-(20)sorbitantrioleat als Tween® 85 im Handel erhältlich, oder Polyoxyethlyensorbithexaoleat als Arlatone®T und G-1086 von Atlas Chemie, oder PEG-7- Glycercocoate, ein Polyoxyethylenglycerinfettsäureester.

Die Zubereitungen können noch weitere nichtethoxylierte und nichtionische Verbindungen enthalten, die u.a. als Emulgatoren oder Netzmittel dienen und deren HLB-Werte etwa im Bereich von 0 bis 9 liegen. Beispiele für solche Verbindungen sind Ester von Glycerin, wie Glycerinmonostearat, oder solche von Sorbitan, wie das Sorbitantrioleat, das beispielsweise als Span®85 im Handel erhältlich ist. Weitere Beispiele sind dazu aus der angegebenen Ullmann-Literatur, Band 22, S. 489, ersichtlich.

Allgemeine Herstellungsbedingungen für die Rezepturbeispiele

Man erwärmt die Fettphasen sowie gegebenenfalls zusätzliche Tenside über den Schmelzpunkt, entgast unter Vakuum und begast mit Stickstoff. Bei Temperaturen unter 50°C werden die Wirkstoffe Dithranol und Harnstoff, als Tensid-Harnstoff-Einschluß-Verbindungen bzw. als Tensid-Harnstoff-"Addukt" zugegeben und mittels Naßmahlwerken feinst dispergiert und danach zügig auf Raumtemperatur abgekühlt. Man erhält leicht verteilbare Öle bzw. Pasten, die nach der therapeutischen Applikation durch Abwaschen mit Wasser leicht und vollständig entfernt werden.

Rezepturbeispiele

## Wasserfreie abwaschbare Massagecreme

| | |
|---|---|
| Myristylalkohol | 40,0 |
| Cocosfettalkoholcaprylat, -caprinat | 15,0 |
| Vaseline, weiß | 28,7 |
| Ceteareth-20 | 4,0 |
| Harnstoff | 12,0 |
| Dithranol | 0,3 |
| | 100,0 |

Ceteareth-20 (4,0) und Harnstoff (12,0): Tensid-Harnstoff- Einschlußverbindung

## Hydrophiles Öl

| | |
|---|---|
| Paraffin, dickflüssig | 80,0 |
| Paraffin, hart | 4,5 |
| Sorbitantrioleat (Span® 85) | 1,5 |
| Polyoxyethylensorbitantrioleat (Tween® 85) | 3,5 |
| Harnstoff | 10,0 |
| Dithranol | 0,5 |
| | 100,0 |

Polyoxyethylensorbitantrioleat (3,5) und Harnstoff (10,0): Tensid-Harnstoff- "Addukt"

<u>Hydrophiles Öl</u>

| | |
|---|---|
| Cetiol® | 31,0 |
| Isopropylmyristat | 24,5 |
| PCL-Liquid (Pur-Zellin-Öl; Fa. Dragoco) | 19,0 |
| Octyldodecanol | 10,0 |
| Polyoxyethylen-Sorbitol-hexaoleat (Arlatone® 1) | 6,0 |
| Polyoxyethylen-Sorbitol-hexaoleat (G.-1086) | 1,5 |
| Harnstoff | 5,0 |
| Dithranol | 3,0 |
| | 100,0 |

6,0 / 1,5 / 5,0 } Tensid-Harnstoff-"Addukt"

<u>Hydrophiles Reinigungsöl</u>

| | | |
|---|---|---|
| Isopropylmyristat | 40,0 | 40,0 |
| Paraffin, flüssig | 20,8 | 14,0 |
| Octyldodecanol | 20,0 | 25,5 |
| Glycerinmonostearat | -- | 3,0 |
| gehärtetes Rizinusöl | 5,0 | -- |
| PEG-7-Glyceryl-cocoate | -- | 5,0 |
| Laureth-2 | 4,0 | -- |
| Harnstoff | 10,0 | 12,0 |
| Dithranol | 0,2 | 0,5 |
| | 100,0 | 100,0 |

4,0 / 10,0 } *

5,0 / 12,0 } **

\* Tensid-Harnstoff-Einschlußverbindung

\*\* Tensid-Harnstoff-"Addukt"

6

**Patentansprüche**

1. Pharmazeutische wasserfreie Zubereitung zur topischen Behandlung von Krankheiten der Haut, insbesondere von Psoriasis, mit Wirkstoffen, die aus Kombination(en) von 1, 8, 9-Anthracentriol und/oder dessen Acylderivaten und Harnstoff bestehen, dadurch gekennzeichnet, daß der Harnstoff in der Zubereitung als feinkristalline Tensid-Harnstoff-Einschlußverbindung bzw. als Tensid-Harnstoff-"Addukt" vorliegt und das Tensid ein nichtionisches Oxethylat mit einer oder mehreren Polyoxyethylengruppen ist.

2. Pharmazeutische wasserfreie Zubereitung nach Anspruch 1, dadurch gekennzeichnet, daß das Tensid in der Tensid-Harnstoff-Einschlußverbindung bzw. in dem Tensid-Harnstoff-"Addukt" eine oxethylierte Verbindung mit einem HLB-Wert zwischen 8 und 15 ist.

3. Pharmazeutische wasserfreie Zubereitung nach den Ansprüchen 1 und 2, dadurch gekennzeichnet, daß diese 1, 8, 9-Anthracentriol und/oder dessen Acylderivate in Mengen von 0,1 bis 5 Gew.-%, insbesondere in Mengen von 0,5 bis 3 Gew.-% und den Harnstoff als Tensid-Harnstoff-Einschlußverbindung bzw. "Addukt" in solchen Mengen, die bei der 1- bis 100-fachen Gewichtsmenge des 1, 8, 9-Anthracentriols und/oder dessen Acylderivaten liegen, enthält.

**Claims**

1. Pharmaceutical anhydrous formulation for topical treatment of diseases of the skin, in particular of psoriasis, containing active ingredients which consist of (a) combination(s) of 1, 8, 9-anthracenetriol and/or acyl derivatives thereof and urea, characterized in that the urea is present in the formulation as a finely crystalline surfactant-urea inclusion compound or as a surfactant-urea "adduct" and the surfactant is a nonionic oxyethylate having one or more polyoxyethylene groups.

2. Pharmaceutical anhydrous formulation according to claim 1, characterized in that the surfactant in the surfactant-urea inclusion compound or in the surfactant-urea "adduct " is an oxyethylated compound having an HLB value of between 8 and 15.

3. Pharmaceutical anhydrous formulation according to claims 1 and 2, characterized in that this contains 1, 8, 9-anthracenetriol and/or acyl derivatives thereof in amounts of 0.1 to 5 wt.%, in particular in amounts of 0.5 to 3 wt.%, and the urea, as a surfactant-urea inclusion compound or "adduct", in amounts which are 1 to 100 times, preferably 2 to 25 times, the amount by weight of the 1, 8, 9-anthracenetriol and/or acyl derivatives thereof.

**Revendications**

1. Préparation pharmaceutique anhydre pour le traitement topique de maladies de la peau, en particulier de psoriasis, contenant des substances actives qui sont constituées par des combinaisons de 1, 8, 9-anthracènetriol et/ou de dérivés acylés de celui-ci et d'urée, caractérisé en ce que l'urée est présente dans la préparation sous forme de composé d'inclusion urée/tenside finement cristallin ou sous forme d'"adduct" urée/tenside, et en ce que le tenside est un oxyéthylate non ionique comportant un ou plusieurs groupements polyoxyéthylène.

2. Préparation pharmaceutique anhydre selon la revendication 1, caractérisée en ce que le tenside dans le composé d'inclusion urée/tenside ou dans l' "adduct" urée/tenside est un composé oxyéthylé ayant une valeur HLB entre 8 et 15.

3. Préparation pharmaceutique anhydre selon la revendication 1 ou 2, caractérisée en ce qu'elle contient du 1, 8, 9-anthracènetriol et/ou des dérivés acylés de celui-ci dans des proportions de 0,1 à 5% en poids, en particulier dans des proportions de 0,5 à 3% en poids, et l'urée sous forme de composé d'inclusion ou d'"adduct" urée/tenside dans des proportions de l'ordre de 1 à 100 fois, de préférance de 3 à 35 fois la quantité en poids du 1, 8, 9-anthracènetriol et/ou des dérivés acylés de celui-ci.